# EUROPEAN PATENT SPECIFICATION

(11) **EP 1 466 586 B1**
(45) Date of publication and mention of the grant of the patent: **12.10.2011**
(21) Application number: 03700531.1
(22) Date of filing: 10.01.2003
(51) Int. Cl.: A61Q 19/00, A61K 8/37, A61K 8/06

(54) **Water-in-oil emulsion preparation for external use on skin**
Wasser-in-Öl-Emulsionszubereitung zur externen Anwendung auf der Haut
Préparation d'une émulsion à phase continue huileuse à usage cutané externe

(30) Priority: 15.01.2002 JP 2002006692
(43) Date of publication of application: 13.10.2004
(73) Proprietor: Nisshin Oillio Group, Ltd., Tokyo 104-8285 (JP)
(72) Inventor: Kachi, Hisanori, Yokohama-shi, Kanagawa 235 (JP); Oyama, Keiichi, Yokohama-shi, Kanagawa 235 (JP)
(74) Representative: Casalonga, Axel
(86) International application number: PCT/JP2003/000168
(87) International publication number: WO 2003/059315

(56) References cited:
- EP-A1- 1 044 672
- DE-A1- 4 444 137
- JP-A- 10 273 433
- JP-A- 2000 086 484
- JP-A- 2001 187 711
- DATABASE CA CHEMICAL ABSTRACTS SERVICE, COLUMBUS, OHIO, US; XP002446429 retrieved from CA Database accession no. 133:155152 & JP 2000 219617 A (SHISEIDO CO., LTD) 28 January 1999 (1999-01-28)
- DATABASE WPI Week 199725 Derwent Publications Ltd., London, GB; AN 1997-276679 XP002446433 & JP 09 100225 A (NOEVIR KK) 15 April 1997 (1997-04-15)
- DATABASE WPI Week 200066 Derwent Publications Ltd., London, GB; AN 2000-674892 XP002446434 & JP 2000 219617 A (SHISEIDO CO LTD) 8 August 2000 (2000-08-08)
- DATABASE WPI Week 198120 Derwent Publications Ltd., London, GB; AN 1981-35392D XP002446435 & JP 56 032408 A (SHISEIDO CO LTD) 1 April 1981 (1981-04-01)

## Description

### TECHNICAL FIELD

This invention relates to a preparation for external use on the skin, and in more detail relates to a water-in-oil emulsion preparation for external use on the skin which can be used as cosmetics, quasi-medicaments, medicaments, etc. and has remarkably enhanced moisture-confining properties, use touch and stability.

### BACKGROUND ART

The object of skin care cosmetics including cream is to supplement moisture and oil to the skin and arrange in a healthy state the skin which has declined, and the most important function of skin care cosmetics is a function to inhibit the transpiration of moisture (hereinafter referred to as moisture-confining properties) for giving the skin moisture and softness. Moisture-confining properties mean such an effect that the thin film of the oil, etc. formed on the skin prevents moisture transpiring from the skin from being released into the air and retains the moisture on the skin, and the larger moisture-confining properties are, the larger the effect to give moisture to the skin is. Cosmetics containing oil and water as basal ingredients are generally classified into water-in-oil emulsions wherein liquid drops as an aqueous phase are dispersed in a continuous phase of the oil and oil-in-water emulsions wherein liquid drops as an oil phase are dispersed in a continuous phase of water, and it is known that water-in-oil emulsions are superior to oil-in-water emulsions in many points such as the protection of the skin and retention of softness.

As to the moisture-confining properties of oil, it has already been reported that nonpolar oils such as petrolatum and liquid paraffin have large moisture-confining properties, and in proportion as the polarity of oil gets higher, its moisture-confining properties gets smaller, as is the case with ester oils. Actually, skin troubles such as rough skin are often caused by dryness of the skin, and it is known that such skin troubles are reduced by effectively inhibiting transpiration of moisture from the skin. Thus, as cosmetics or external preparations having large moisture-confining properties, ones wherein petrolatum is compounded are on the market.

However, since nonpolar oils such as petrolatum and liquid paraffin generally do not have good affinity to sebum, they slide on the skin after being applied onto it, and not a few of complaints have been made that when creams, etc. containing a nonpolar oil as a main ingredient are applied onto a hand, it gets hard to hold a pen firmly with the hand because of slip, the oil moves onto clothes, paper, etc. touched with the hand, and so on. Furthermore, in the case of cosmetics such as foundations wherein powder is compounded into a nonpolar oil, there have been problems that when the skin coated with such a cosmetic touched clothes, the powder easily moves onto the clothes.

On the other hand, in preparations using a water-in-oil emulsion, it is comparatively difficult to retain their emulsion state over a long period. As methods which have hitherto been carried out for improving the emulsion stability, there is a method in which a large amount of a wax is compounded into the oil phase as the continuous phase to heighten the viscosity of the emulsion, but even in this method, phenomena such as softening and fusion of the compounded wax may occur depending on various temperature changes and its emulsion stability was not adequate. Further in the point of use touch, there were problems that the emulsion is sticky, oily, only poorly spreads, and so on, due to the wax.

Ester oils, as polar oils, including isooctyl palmitate, neopentyl glycol dicaprare, glycerol tri-2-ethylhexanoate, etc. are generally superior to hydrocarbon oils such as petrolatum, liquid paraffin and part of waxes in miscibility with sebum, and thus have such advantages that they do not slide on the skin, have good affinity to the skin, are hard to shine the skin, and so on; and, moreover, ester oils are excellent in air permeability and have less oily touch; and, therefore, ester oils are commonly used in cosmetics using an oil. Furthermore, since cosmetics in which an ester oil is compounded have good affinity to the skin, there seldom arise such problems, as in cosmetics in which petrolatum is compounded, that the oil moves onto clothes, etc. ,and so on.

However, since ester oils are rich in air permeability, they are much inferior to petrolatum in moisture-confining properties, and water-in-oil emulsion cosmetics containing an ester oil as a main ingredient are not adequate in moisture-confining properties which are a main object of oil, and their effect to improve rough skin, etc. has been poor. Furthermore, when an ester oil is compounded into a conventional water-in-oil emulsion, phase separation, etc. tend to be caused, and the obtained emulsion has a Problem in emulsion stability. Thus, although ester oils are excellent in use touch, there are many restrictions about their compounding, and there have been few cases where an amount of an ester oil enough to actually improve use touch is used.

In recent years, concern for influence of ultraviolet rays on the skin has been increasing, and a demand for sunscreening cosmetics is increasing. Commercial sunscreening cosmetics are generally and typically water-in-oil emulsion cosmetics, and, in almost all of them, an ultraviolet absorbing oil is compounded for heightening the value of SPF (Sun Protection Factor), and an ultraviolet absorber as an ester oil such as, for example, a paramethoxycinnamic ester or an N,N-dimethylparaaminobenzoic ester is frequently used. However, since these ester oils having an ultraviolet absorbing functional group have especially high polarity, it is difficult to emulsify them and maintain their stability, and, nevertheless, for preparing such an water-in-oil emulsion composition, it is compelled to compound a large amount of a wax or use waxes in a combination, and, after all, it has been frequent that it cannot help limiting the compounding amount of the ester oil.

Under these circumstances, there has been desired a water-in-oil emulsion preparation for external use on the skin which has good affinity to the skin of an ester oil while it makes moisture-confining properties equal to petrolatum come true, and, in addition, which is excellent in emulsion stability.

It is known that some of oily substances obtained by esterifying a polyhydric alcohol, a medium-chain or long-chain fatty acid, and a medium-chian dibasic acid are compounded into lipsticks or oil-in water creams since they have good adhesion and, when they are used as an oil ingredient of oil-in water compositions which is emulsified, contribute their emulsion stability From JP 2000-219617 is known an ester compound obtained by esterifying (1) a polyhydric alcohol, (2) a straight-chain, branched, unsaturated or hydroxy-fatty acid having 8 to 30 carbon atoms and (3) a straight-chain or branched dibasic acid having 12 to 30 carbon atoms, in a sunscreen cosmetic composition comprising an ultraviolet absorber.

### DISCLOSURE OF INVENTION

In such state of things, this invention aims to provide a water-in-oil emulsion preparation for external use on the skin which solves the above problems, and is excellent in moisture-confining properties, good in use touch and, further, excellent in emulsion stability.

The present inventors, in the state of affairs, intensely studied for obtaining a water-in-oil emulsion preparation for external use on the skin having good moisture-confining properties, good stability and good use touch, and as a result they found that the above object can be attained by compounding a specific ester compound together with an ester oil into the oil phase and compounding a water soluble inorganic salt into the aqueous phase, and completed the present invention.

Namely, the invention relates to a water-in-oil emulsion preparation for external use on the skin, imparting moisture-confining properties, use touch and emulsion stability, wherein the oil phase comprises a mixture of an ester compound obtained by esterifying (A) a polyhydric alcohol, (B) a fatty acid and/or hydroxy fatty acid each having 8 to 30 carbon atoms and (C) a dibasic carboxylic acid having 12 to 30 carbon atoms; an ester oil; a surfactant; and an oil other than ester oil, and the aqueous phase comprises an aqueous solution of a water-soluble inorganic salt, the compounding amounts of the ester oil in the oil phase is 20 to 75 % by mass based on the total mass of the ester compound, the ester oil, the surfactant and the oil other than ester oil, and the water-soluble inorganic salt is at least one selected from sodium chloride, potassium chloride, magnesium chloride, sodium sulfate, sodium phosphate, disodium hydrogenphosphate, sodium dihydrogenphosphate, potassium phosphate, dipotassium hydrogenphosphate and potassium dihydrogenphosphate.

### BEST MODE FOR CARRYING OUT THE INVENTION

The invention is described in detail below.

The oil phase of the water-in-oil emulsion preparation for external use on the skin of the invention, basically, comprises the above ester compound, an ester oil, a surfactant for emulsification and an oil other than ester oil (solid fat or oil, wax or the like).

The ester compound used in the invention is an ester compound obtained by esterifying (A) a polyhydric alcohol, (B) a fatty acid and/or hydroxy fatty acid each having 8 to 30 carbon atoms and (C) a dibasic carboxylic acid having 12 to 30 carbon atoms. Mixing proportion among these ingredients is not particularly limited, but is preferably ingredient (A) : ingredient (B) : ingredient (C) = 1.0 mole : 1

> 1.0 to 2.5 moles : 0.25 to 1.0 mole. There is no particular limitation about the esterification method, and a known esterification method can be used. The resulting ester compound is a mixture of ester compounds which are mutually different in contents of the residue of ingredient (A), the residue of ingredient (B) and the residue of ingredient (C), and it can, usually, be used as such, namely as the mixture.

The ester compound in the oil phase of the water-in-oil emulsion preparation for external use on the skin of the invention has a function to make the oils in the oil phase, namely the ester oil and the oil other than ester oil gelate, and it is considered that, by this function, the use touch of the water-in-oil emulsion preparation for external use on the skin is heightened, and the dispersion stability of the aqueous phase is heightened. In other words, it is considered that the ester compound stabilizes the dispersion of the aqueous phase, and further heightens the use touch of the water-in-oil emulsion preparation for external use on the skin, by making the oils form a uniform gel and confining the aqueous phase in the gel in a dispersed state without separating it.

As the polyhydric alcohol which is a component of the ester compound used in the invention, ones having three or more, particularly 3 to 6 hydroxyl groups are preferred, and, there can, for example, be mentioned glycerol, glycerol condensates, trimethylolpropane, pentaerythritol, trimethylolethane, sorbitol, xylitol, sucrose, glucose, fructose, mannitol, etc.

The fatty acid and/or hydroxy fatty acid each having 8 to 30 carbon atoms which are/is component(s) of the ester compound used in the invention is such that the hydrocarbonic group and/or hydroxy hydrocarbonic group may be straight-chain or branched-chain and may be saturated or unsaturated, and there can, for example, be mentioned caprylic acid, capric acid, lauric acid, myristic acid, palmitic acid, stearic acid, behenic acid, oleic acid, ricinolic acid, palmitoleic acid, isooctylic acid (2-ethylhexanoic acid, etc.), isononanoic acid (3,5,5-trimethylhexanoic acid, etc.), isopalmitic acid, isostearic acid (2-heptylundecanoic acid, multimethyl branched type made by Emery Corporation, etc.), cerotic acid, montanic acid, melissic acid, etc. For still further heightening the long-term stability of the water-in-oil emulsion preparation for external use on the skin, it is desirable to use one or more of straight-chain, long-chain saturated fatty acids having 16 to 28 carbon atoms such as, particularly, palmitic acid, stearic acid, behenic acid, cerotic acid and montanic acid. Fatty acids having 7 or less carbon atoms are improper in the points of skin-stimulating properties and/or stability against hydrolysis. When the carbon number is 31 or more, the esterification reaction does not proceed smoothly, and it gets hard to obtain the desired ester compound used in the invention.

The dibasic carboxylic acid having 12 to 30 carbon atoms which is a component of the ester compound used in the invention may be straight-chain or branched-chain and may be saturated or unsaturated, but is preferably saturated, and there can, for example, be mentioned eicosanedioic acid, dodecanedioic acid, 1,10-decamethylenedioic acid, 1,12-dodecamethylenedioic acid, 1,15-pentadecamethylenedioic acid, 1,28 -octacosamethylenedioic acid, 1,7-ethyloctadecanedioic acid, etc.

When a dibasic carboxylic acid having 11 or less carbon atoms is used, even if an aqueous phase is added to an oil phase containing the resulting ester compound as an ingredient and the mixture is subjected to emulsification to obtain a water-in-oil emulsion, adequate thickening effect cannot be obtained, separation of the aqueous phase occurs soon, and thus, a stable emulsion composition cannot be obtained. The reason is presumed to be that a tendency for the ester compound to get not to show a characteristic to make the oils gelate in the oil phase gets larger. On the other hand, when a dibasic carboxylic acid having 31 or more carbon atoms is used, it gets hard to make the esterification reaction proceed, and physical properties of the resulting product gets not stable. From the above viewpoint, the carbon number of the dibasic carboxylic acid is preferably 14 to 28, and more preferably 16 to 28.

In the invention, it is particularly preferred, in view of the use touch and stability over day's lapse of the water-in-oil emulsion preparation for external use on the skin, to use, as the ester compound, glycerol behenate eicosanedioate which is an ester compound obtained by esterifying glycerol, behenic acid and eicosanedioic acid.

It is necessary to compound the ester compound in the oil phase in an amount of 0.1 to 30 % by mass, based on the total mass of the ester compound, the ester oil, the surfactant and the oil other than ester oil, and its compounding amount is preferably 0.5 to 25 % by mass and more preferably 1 to 20 %. When the compounding amount is less than 0.1 % by mass, sufficient effect in the stability and use touch of the resulting preparation cannot be obtained. When it is more than 30 % by mass, spread of the preparation goes poor and use touch is spoiled.

It is also considered that the ester compound, after being applied onto the skin, forms an oil film having appropriate moisture permeability and controls moisture retention. There is amorphous microcrystalline wax as a wax often compounded in water-in-oil emulsion cosmetics for the purpose of stabilization of emusification, but the ester compound used in the invention is superior thereto in moisture-confining properties and has moisture-confining properties almost equal to petrolatum, and, therefore, it is expected that a water-in-oil emulsion preparation for external use on the skin prepared by compounding the ester compound into an oil phase has excellent moisture retention. Cosmetics, etc. in which an ester oil is compounded as a main ingredient of the oil phase are sometimes unsatisfactory in moisture-confining properties after being applied, but such problem can be solved by compounding such an ester compound. On the other hand, since the ester compound has many ester bonds, it is excellent in affinity with sebum and has only small oily stickiness, when the hands coated with the preparation of the invention touched clothes or paper, it is possible to prevent the oil ingredients from moving onto them. It is therefore considered that the water-in-oil emulsion preparation for external use on the skin of the invention is free from stickiness as in the case where stabilization was made by compounding wax, but, nevertheless, gives good moisturizing touch as if there were neither too much nor too little moisture on the skin.

An ester compound, microcrystalline wax and petrolatum were actually checked for moisture retention, and the results are shown below for reference. The moisture permeability of the oils and the ester compound was evaluated according to the method of JIS Z0208 "Method of Testing Damp Proofing Packaging Materials for Moisture Permeability (Cup Method)" by the amount of moisture which permeated through an oil film prepared so as to have the thickness of filter paper. Moisture-permeable cups of a diameter of 60 mm each having a moisture-permeable surface at the top part were used, and 20 g of glycerol was used as a moisture absorbent, and beeswax: paraffin wax (=60:40) as a sealing wax. Filter paper of 5C was impregnated with a definite amount (7 to 9 mg/cm²) of a sample shown in Table 1 to form a film with a definite thickness. The resulting filter paper was placed on the moisture-permeable cup containing 20 g of glycerol, and sealing was made using the sealing wax, and the mass of whole cup was measured. An atmosphere of high humidity (25°C, 80%) was produced using aqueous saturated ammonium chloride solution, and the moisture-permeable cup was allowed to stand for 3 hours in the atmosphere, and then the mass of the cup was measured. As a blank test, filter paper of 5C not impregnated with a sample was used, and the mass of moisture which permeated (moisture permeation amount) through the filter paper was measured, and the case of the moisture permeation amount is assumed to be a moisture permeability of 100%. The moisture permeability of each sample was expressed as the percentage of a value obtained by dividing the difference in mass between before and after the resulting cup was allowed to stand under the high humidity (moisture permeation amount) by the moisture permeation amount in the blank test. The results are shown in Table 1.

**Table 1**

| Sample No. | 1 | 2 | 3 | 4 | 5 |
|---|---|---|---|---|---|
| Oil A (% by mass) | None | T.I.O 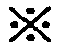 1 (100%) | T.I.O (90%) | T.I.O (90%) | Petrolatum (100%) |
| Oil A (% by mass) | None | None | Ester compound 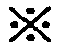 2 (10%) | Microcrystalline wax (10%) | None |
| Moisture Permeation amount | 0.270g | 0.057g | 0.015g | 0.024g | 0.009g |
| Moisture permeability | 100% | 21.1% | 5.6% | 8.9% | 3.3% |
| Note | Blank test | | | | |

| | | | | | |
|---|---|---|---|---|---|
| 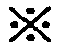 1 Glycerol tri-2-ethylhexanoate 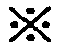 2 Glycerol behenate eicosanedioate (trade name: NOMCORT HK-G, made by THE NISSHIN OIL MILLS, LTD.) | | | | | |

As the ester oil compounded into the oil phase of the water-in-oil emulsion preparation for external use on the skin of the invention, ester oils usually usable for cosmetics, etc. can be used. As such ester oils, there can, for example, be mentioned isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, octyldodecyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerol di-2-heptylundecanoate, trimethylolpropane tri-2-ethylhexanoate, trimethylolpropane triisostearate, pentaerythritol tetra-2-ethylhexanoate, glycerol tri-2-ethylhexanoate, glycerol tri(caprylate, caprate, myristate and stearate), trimethylolpropane triisostearate, cetyl 2-ethylhexanoate, 2-ethylhexyl palmitate, glycerol trimyristate, glycerol tri-2-heptylundecanoate, methyl esters of castor oil fatty acids, oleyl oleate, glycerol acetate, 2-heptylundecyl palmitate, diisobutyl adipate, (adipic acid, 2-ethylhexanoic acid and stearic acid) oligoesters of glycerol, (2-hexyldecanoic acid and sebacic acid) oligoesters of diglycerol, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, avocado oil, camellia oil, turtle oil, macadamia nut oil, corn oil, sunflower seed oil, mink oil, olive oil, rapeseed oil, egg yolk oil, sesame oil, persic oil, wheat germ oil, sasanqua oil, castor oil, linseed oil, safflower oil, grapeseed oil, cotton seed oil, perilla oil, soybean oil, peanut oil, tea seed oil, kaya oil, rice germ oil, Japanese tung oil, jojoba oil, germ oil, evening primrose oil, glycerol trioctanoate, glycerol triisopalmitate, ethyl acetate, butyl acetate, triethyl citrate, etc.

Among ester oils, ones containing a branched fatty acid and/or a branched alcohol as a constituent are called branched ester oils. Among ester oils, branched ester oils show better affinity to the skin, but show lower moisture-confining properties. In the invention, since the ester compound improves the lowness of moisture-confining properties which is a defect of ester oils, even if a branched ester oil is compounded, it is possible to retain adequate moisture-confining properties and, at the same time, reinforce good affinity to the skin.

The compounding amount of the ester oil into the oil phase of the water-in-oil emulsion preparation for external use on the skin of the invention is not particularly limited, so long as it is an amount enough to give good use touch such as good spread and good affinity to the skin when the preparation is applied to the skin, and is hard to uniformly prescribe, but, usually, is 20 to 75 % by mass, based on the total mass of the ester compound, the ester oil, the surfactant and the oil other than ester oil.

The use ratio of the ester compound to the ester oil in the oil phase of the water-in-oil emulsion preparation for external use on the skin of the invention is preferably 1:99 to 95:5, more preferably 5:95 to 95:5, and still more preferably 10:90 to 90:10. When the use ratio of the ester compound is less than 1:99, moisture-confining properties generally tend to be inadequate, and, moreover, there occurs a tendency that phase separation arises and emulsion stability is spoiled. When the use ratio of the ester compound is more than 95:5, there occurs a tendency that when the resulting preparation is applied onto the skin, spread is poor and touch is heavy.

Various surfactants have hitherto been used for obtaining water-in-oil emulsion preparations for external use on the skin. Also in the invention, it is possible to use one or two or more in combination of synthetic surfactants and/or natural surfactants such as anionic surfactants, cationic surfactants, amphoteric surfactants, lipophilic nonionic surfactants and hydrophilic nonionic surfactants. In the invention, the stability of the water-in-oil emulsion is heightened by the ester compound and the water soluble inorganic salt contained in the oil phase and the aqueous phase, respectively, and the kind of the surfactant used as an emulsifier is not limited, but there can be mentioned following ones as examples.

As anionic surfactants, there can, for example, be mentioned fatty acid soaps such as Soap Base, sodium laurate and sodium palmitate; salts of higher alkyl sulfate esters such as sodium lauryl sulfate and potassium lauryl sulfate; salts of alkyl ether sulfate esters such as triethanolamine salts of POE-lauryl sulfate and sodium POE-lauryl sulfate; N-acylsarcosines such as sodium lauroylsarcosine; salts of higher fatty acid amidosulfonic acids such as sodium N-myristoyl-N-methyl-taurine, Sodium N-Cocoyl-N-Methyl Taurate and sodium lauroylmethyl taurate; salts of phosphoric esters such as sodium POE-oleyl ether phosphate and sodium POE-stearyl ether phosphate; saltes of sulfosuccinic esters such as sodium di-2-ethylhexyl sulfosuccinate, sodium monolauroyl monoethanolamidopolyoxyethylenesulfosuccinate and sodium lauryl polypropylene glycol sulfosuccinate; salts of alkylbenzenesulfonic acids such as sodium linear dodecylzenesulfonate, triethanolamine salt of linear dodecylzenesulfonate and sodium linear dodecylzenesulfonate; salts of N-acylglutamic acid such as monosodium N-lauroylglutamate, disodium N-stearoylglutamate and monosodium N-myristoyl-L-glutamate; salts of higher fatty acid and sulfuric acid esters of glycerol such as sadium salt of hardened coconut oil fatty acid ester and sulfuric ester of glycerol; sulfated oils such as Turkey red oil; salts of POE-alkyl ether carboxylic acids; salts of POE-alkylaryl ether carboxylic acids; salts of α -olefinsulfonic acids; salts of higher fatty acid ester sulfonic acids; salts of secondary alcohol sulfate esters; salts of higher fatty acid alkylolamide sulfate esters; sodium lauroyl monoethanolamidosuccinate; di-triethanolamine salt of N-palmitoylaspartic acid; sodium caseinate, etc.

As cationic surfactants, there can, for example, be mentioned alkyltrimethylammonium salts such as stearyltrimethylammonium chloride and lauryltrimethylammonium chloride; dialkyldimethylammonium salts such as distearyldimethylammonium chloride; poly(N,N'-dimethyl-3,5-methylenepiperidinium) chloride; alkylpyridinium salts such as cetylpyridinium chloride; alkyltetraammonium salts; alkyldimethylbenzylammonium salts; alkylisoquinolinium salts; dialkylmorpholinium salts; POE-alkylamines; alkylamine salts; polyamine fatty acid derivatives; amyl alcohol fatty acid derivatives; benzalkonium chloride; benzethonium chloride; organically modified clay minerals such as organically modified montmorillonite; etc.

As amphoteric surfactants, there can, for example, be mentioned imidazoline-type amphoteric surfactants such as sodium 2-undecyl-N,N,N-(hydroxyethylcarboxymethyl)-2-imidazoline and disodium 2-cocoyl-2-imidazolinium hydroxide-1-carboxyethyloxy; betaine-type surfactants such as 2-heptadecyl-N-carboxymethyl-N-hydroxyethylimidazolinium betaine, lauryldimethylaminoacetic acid betaine, alkyl betaines, amide betaines and sulfobetaines; etc.

As lipophilic nonionic surfactants, there can, for example, be mentioned sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerolsorbitan penta-2-ethylhexylate and diglycerolsorbitan tetra-2-ethylhexylate; glycerol fatty acid esters such as glycerol mono-cotton seed oil fatty acid ester, glycerol monoerucate, glycerol sesquioleate, glycerol monostearate, glycerol α, α'-oleate pyroglutamate, glycerol monostearate and glycerol monooleate; polyglycerol fatty acid esters such as diglycerol monoisostearate, diglycerol diisostearate, diglycerol condensed licinolate and tetraglycerol condensed licinolate; propylene glycol fatty acid esters such as propylene glycol monostearate; hardened castor oil derivatives; glycerol alkyl ethers; etc.

As hydrophilic nonionic surfactants, there can, for example, be mentioned POE-sorbitan fatty acid esters such as POE-sorbitan monooleate, POE-sorbitan monostearate and POE-sorbitan tetraoleate, POE-sorbitol fatty acid esters such as POE-sorbitol monolaurate, POE-sorbitol monooleate, POE-sorbitol pentaoleate and POE-sorbitol monostearate; POE-glycerol fatty acid esters such as POE-glycerol monostearate, POE-glycerol monoisostearate and POE-glycerol triisostearate; POE fatty acid esters such as POE monooleate, POE distearate, POE dioleate and ethylene glycol distearate; POE alkyl ethers such as POE lauryl ether, POE oleyl ether, POE stearyl ether, POE behenyl ether, POE 2-octyldodecyl ether and POE cholestanol ether; Pluronics such as Pluronic; POE.POP alkyl ethers such as POE.POP cetyl ether, POE.POP 2-decyltetradecyl ether, POE.POP monobutyl ether, POE.POP hydrogenated lanolin ether and POE.POP glycerol ether; tetraPOE.tetraPOP ethylenediamine condensates such as Tetraronic; POE-castor oil / hardened castor oil derivatives such as POE-castor oil, POE-hardened castor oil, POE-hardened castor oil monoisostearate, POE-hardened castor oil triisostearate, POE-hardened castor oil monopyroglutamate monoisostearate diester and POE-hardened castor oil maleate; POE-beeswax lanolin derivatives such as POE-sorbitol beeswax; fatty acid alkanolamides such as coconut oil fatty acid diethanolamide, lauric acid monoethanolamide and fatty acid isopropanolamides; POE-propylene glycol fatty acid esters; POE-alkylamines; POE-fatty acid amides; sucrose fatty acid esters; POE-nonylphenyl formaldehyde condensates; alkylethoxydimethylamine oxides; trioleyl phosphate; polyglycerol fatty acid esters such as polyglycerol monolaurate, polyglycerol monostearate, polyglycerol monooleate, polyglycerol distearate and polyglycerol dioleate; modified silicones such as copolymers of methylpolysiloxane, cetylmethylpolysiloxane and poly(oxyethylene.oxypropylene)methylpolysiloxane; etc.

As natural surfactants, there can, for example, be mentioned lecithins such as soybean phospholipid, hydrogenated soybean phospholipid, yolk phospholipid and hydrogenated yolk phospholipid; soybean saponin; etc.

The compounding amount of the surfactant into the oil phase is not particularly limited so long as a stable water-in-oil emulsion preparation for external use on the skin can be obtained, but, usually, it is preferably 0.01 to 40 % by mass, more preferably 0.05 to 30 % by mass, still more preferably 0.1 to 20 % by mass, based on the total mass of the ester compound, the ester oil, the surfactant and the oil other than ester oil.

The oil other than ester oil constructing the oil phase of the water-in-oil emulsion preparation for external use on the skin of the invention is not particularly limited, and, among various oils usually used for cosmetics or external preparations, oils other than ester oils can be used, and, there can, for example, be mentioned solid fats and oils, waxes, hydrocarbon oils, higher fatty acids, higher alcohols, silicone oils, etc. Specific examples of the oil other than ester oil are given below.

As the solid fats and oils, there can, for example, be mentioned cacao butter, coconut oil, tallow, mutton tallow, equine fat, palm kernel oil, lard, bovine bone fat, Japan kernel oil, bovine leg fat, Japan wax, hardened coconut oil, hardened palm oil, hardened tallow, hardened oil, hardened castor oil, etc.

As the waxes, there can, for example, be mentioned beeswax, candelilla wax, cotton wax, carnauba wax, bayberry wax, insect wax, spermaceti, montan wax, rice bran wax, kapok wax, sugar cane wax, lanolin, lanolin acetate, liquid lanolin, isopropyl lanolate, reduced lanolin, hard lanolin, hexyl laurate, jojoba wax, shellac wax, POE lanolin alcohol ether, POE lanolin alcohol acetate, POE cholesterol ether, polyethylene glycol lanolate, POE hydrogenated lanolin alcohol ether, etc.

As the hydrocarbon oils, there can, for example, be mentioned liquid paraffin, isoparaffins, paraffins, ozocerite, squalane, pristane, ceresin, squalene, Vaseline, microcrystalline wax, paraffin wax, α-olefin oligomers, etc.

As the higher fatty acids, there can, for example, be mentioned lauric acid, myristic acid, palmitic acid, stearic acid, behenic acid, oleic acid, undecylenic acid, cholic acid, isostearic acid, linolic acid, linolenic acid, eicosapentaenoic acid (EPA), docosahexaenoic acid (DHA), etc.

As the higher alcohols, there can, for example, be mentioned straight-chain alcohols such as lauryl alcohol, cetyl alcohol, stearyl alcohol, behenyl alcohol, myristyl alcohol, oleyl alcohol and cetostearyl alcohol; branched-chain alcohols such as monosteary ether of glycerol (batyl alcohol), 2-decyltetradecanol, lanolin alcohol, cholesterol, phytosterol, hexyldodecanol, isostearyl alcohol and octyldodecanol; etc.

As the silicone oils, there can, for example, be mentioned chain polysiloxanes such as dimethylpolysiloxane, methylphenylpolysiloxane and methylhydrogenpolysiloxane; cyclic polysiloxanes such as octamethylcyclotetrasiloxane, decamethyl cyclopentasiloxane, dodecamethylcyclohexasiloxane and tetrahydrotetramethylcyclotetrasiloxane; polyoxyethylenepolyalkylsiloxane; etc.

The content of the oil other than ester oil in the oil phase of the water-in-oil emulsion preparation for external use on the skin of the invention is not particularly limited, but, usually, is preferably 0.01 to 98 % by mass, based on the total mass of the ester compound, the ester oil, the surfactant and the oil other than ester oil.

The aqueous phase of the water-in-oil emulsion preparation for external use on the skin of the invention is, basically, constructed from a water soluble inorganic salt and water. As the water soluble inorganic salt incorporated into the aqueous phase, there can be mentioned at least one selected from sodium chloride, potassium chloride, magnesium chloride, sodium sulfate, sodium phosphate, disodium hydrogenphosphate, sodium dihydrogenphosphate, potassium phosphate, dipotassium hydrogenphosphate and potassium dihydrogenphosphate.>

Particularly preferred are sodium chloride, potassium chloride and sodium sulfate. These can be used alone or as a mixture of an arbitrary proportion. It is necessary for the compounding amount of the water soluble inorganic salt into the aqueous phase to be 0.01 to 10% by mass, based on the total mass of the water soluble inorganic salt and water, and the amount is preferably 0.1 to 5% by mass, and more preferably 0.5 to 5% by mass.

It is presumed that, by incorporating a water soluble inorganic salt in the aqueous phase, the inorganic salt controls transpiration and absorption of moisture on the skin. By this, it is possible to obtain a water-in-oil emulsion preparation for external use on the skin which has more sustained moisturizing touch after its application, in addition to moisture-confining properties reinforced by the ester compound.

When such a water soluble inorganic salt is not added to the aqueous phase, a stable water-in-oil emulsion composition cannot be formed, and 2 to 3 days after the preparation of the emulsion composition, separation of the aqueous phase takes place. When the addition amount of the water soluble inorganic salt to the aqueous phase is less than 0.01% by mass, based on the total mass of the water soluble inorganic salt and the water, a similar tendency is observed, and although emulsion stability over the lapse of time is somewhat better compared with the case of no addition, separation of the aqueous phase occurs before long. By the addition of 0.01 to 10% by mass, above all 0.1 to 5% by mass, the above problem is obviated. On the other hand, when the water soluble inorganic salt is compounded in an amount of more than 10% by mass into the aqueous phase, the resulting emulsion composition gives sticky touch to the skin when applied thereon.

The ratio by mass of the oil phase to the aqueous phase in the water-in-oil emulsion preparation for external use on the skin of the invention is preferably 10:90 to 90:10, more preferably 20:80 to 80:20, and still more preferably 30:70 to 70:30. When the proportion of the aqueous phase is larger than the ratio by mass of 10:90, preservation stability tends to go bad. When the proportion of the aqueous phase is smaller than the ratio by mass of 90:10, sticky touch due to the oils gets stronger, which is undesirable in view of use touch.

If desired, it is possible to compound, into the water-in-oil emulsion preparation for external use on the skin of the invention, in addition to the above-mentioned ingredients, various ingredients for further improving use touch, further giving moisturizing properties, giving nutrition to the skin, preventing deterioration of quality, and/or so on, which various ingredients are used in general in cosmetics, medicaments, etc. (hereinafter referred to as physical properties, etc.-improving agents) in an amount not to spoil the effects of the invention. As such physical properties, etc.-improving agents, there can, for example, be mentioned powder ingredients, humectants, natural water soluble macromolecules, semi-synthetic water soluble macromolecules, synthetic water soluble macromolecules, inorganic water soluble macromolecules, ultraviolet absorbers, sequestering agents, lower alcohols, polyhydric alcohols, monosaccharides, oligosaccharides, polysaccharides, amino acids, organic amines, synthetic resin emulsions, pH-adjusting agents, vitamins, antioxidants, antioxidant's assistants, perfumes, etc. These can be used alone or in combination of two or more.

As the powder ingredients, inorganic powders such as talc, kaolin, mica, sericite, muscovite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, metallic salts of tungstic acid, magnesium, silica, zeolite, barium sulfate, calcined calcium sulfate (calcined gypsum), calcium phosphate, fluorapatite, hydroxyapatite, ceramic powder, metallic soaps (zinc myristate, calcium palmitate, aluminum stearate) and boron nitride; organic powders such as polyamide resin powder (nylon powder), polyethylene powder, polymethyl methacrylate powder, polystyrene powder, powder of copolymer resin of styrene and acrylic acid, benzoguanamine resin powder, poly ethylene tetrafluoride powder and cellulose powder; inorganic white pigments such as titanium dioxide and zinc oxide; inorganic red pigments such as iron oxide (red oxide) and iron titanate; inorganic brown pigments such as γ-iron oxide; inorganic yellow pigments such as yellow iron oxide and loess; inorganic black pigments such as black iron oxide, carbon black and titanium oxide of a low oxidation extent; inorganic purple pigments such as ammonium manganese pyrophosphate and cobalt violet; inorganic green pigments such as chromium oxide, chromium hydroxide and cobalt titanate; inorganic blue pigments such as ultramarine blue and prussian blue; pearlescent pigments such as mica coated with titanium oxide, bismuth oxychloride coated with titanium oxide, talc coated with titanium oxide, mica coated with colored titanium oxide, bismuth oxychloride and fish scale guanine; metal powder pigments such as aluminum powder and copper powder; organic pigments such as Red 201, Red 202, Red 204, Red 205, Red 220, Red 226, Red 228, Red405, Orange 203, Orange 204, Yellow 205, Yellow 401 and Blue 404; organic pigments including zirconium lakes, barium lakes, aluminum lakes, etc. such as, for example, Red 3, Red 104, Red 106, Red 227, Red 230, Red 401, Red 505, Orange 205, Yellow 4, Yellow 5, Yellow 202, Yellow 203, Green 3 and Blue 1; natural coloring matters such as chlorophyll and β-carotene; etc. But, the powder ingredients include powders usable for general cosmetics, and are not limited to the ones enumerated above.

As the humectants, there can, for example, be mentioned polyethylene glycol, propylene glycol, glycerol, 1,3-butylene glycol, xylitol, sorbitol, maltitol, chondroitin sulfuric acid, hyaluronic acid, mucoitin sulfuric acid, charonin sulfuric acid, atelocollagen, cholesteryl 12-hydroxystearate, sodium lactate, urea, salts of bile acid, salts of dl-pyrrolidonecarboxylic acid, short-chain soluble collagen, diglycerol (EO) PO adducts, extract of hestnut rose, extract of yarrow, extract of sweet clover, etc.

As the natural water soluble macromolecules, there can, for example, be mentioned vegetable macromolecules such as gum arabic, tragacanth gum, galactan, guar gum, karaya gum, carrageenan, Locust bean gum, tamarind seed gum, pectin, agar, quince seed (Cydonia vulgalis Peas), algae colloid (marine plants extract) and starch (rice, corn, potato, wheat); microbial macromolecules such as xanthane gum, dextran, succinylglucans and pullulan; animal macromolecules such as collagen, casein, albumin and gelatin.

As the semi-synthetic water soluble macromolecules, there can, for example, be mentioned starch-type macromolecules such as carboxymethylstarch and methylhydroxypropylstarch; cellulos-type macromolecules such as methylcellulose, nitrocellulose, methylhydroxypropylcellulose, sodium cellulose sulfate, hydroxypropylcellulose, carboxymethylcellulose, sodium carboxymethylcellulose, crystalline cellulose and cellulose powder; alginic acid-type macromolecules such as sodium alginate and propylene glycol alginate; etc.

As the synthetic water soluble macromolecules, there can, for example, be mentioned vinyl-type macromolecules such as polyvinyl alcohol, polyvinyl methyl ether, polyvinylpyrrolidone and carboxyvinyl polymers (carbopol); polyoxyethylene-type macromolecules such as polyethylene glycols 20,000, 40,000 and 60,000; polyoxyethylene-polyoxypropylene copolymer-type macromolecules; acrylic-type macromolecules such as sodium polyacrylate, polyethyl acrylate and polyacrylamide; polyethylene-imine; cationic polymers, etc.

As the inorganic water soluble macromolecules, there can, for example, be mentioned bentonite, AlMg silicate (veegum), hectorite, silicic acid anhydride, etc.

As the ultraviolet absorbers, there can, for example, be mentioned benzoic acid-type ultraviolet absorbers such as paraaminobenzoic acid (hereinafter abbreviated as PABA), glycerol monoPABA ester, N,N-dipropoxyPABA ethyl ester, N,N-diethoxyPABA ethyl ester, N,N-dimethylPABA ethyl ester and N,N-dimethyl PABA butyl ester; anthranilic acid-type ultraviolet absorbers such as homomenthyl N-acetylanthranilate; salicylic acid-type ultraviolet absorbers such as amyl salicylate, menthyl salicylate, homomenthyl salicylate, octyl salicylate, phenyl salicylate, benzyl salicylate and p-isopropylphenyl salicylate; cinnamic acid-type ultraviolet absorbers such as octyl cinnamate, ethyl 4-isopropylcinnamate, methyl 2,5-diisopropylcinnamate, ethyl 2,4 -diisopropylcinnamate, methyl 2,4 -diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate and glycerol mono-2-ethylhexanoyl-diparamethoxycinnamate; benzophenone-type ultraviolet absorbers such as 2,4-dihydroxybenzophenone, 2,2' -dihydroxy-4-methoxybenzophenone, 2,2' -dihydroxy-4,4'-dimethoxybenzophenone, 2,2' ,4,4'-tetrahydroxybenzophenone, 2-hydroxy-4-methoxybenzophenone, 2-hydroxy-4-methoxy-4'-methylbenzophenone, salts of 2-hydroxy-4-methoxybenzophenone-5-sulfonic acid, 4-phenylbenzophenone, 2-ethylhexyl 4'-phenylbenzophenone-2-carboxylate, 2-hydroxy-4-n-octoxybenzophenone and 4-hydroxy-3-carboxybenzophenone; 3-(4'-methylbenzyhdene)-d,1-camphor; 3-benzylidene-d,1-camphor; urocanic acid; ethyl urocanate; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dianisoylmethane; 4-methoxy-4'-t-butyldibenzoylmethane; 5-(3,3-dimethyl-2-norbornylidene)-3-pentan-2-one; 2,4,6-trianilino-p-(carbo-2'-ethylhexyl-1'-oxy)-1,3,5-triazine; etc. Many of them (for example, benzoic acid-type, anthranilic acid-type, salicylic acid-type, cinnamic acid-type, etc.) are ester oils.

As the sequestering agents, there can, for example, be mentioned 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium ethylenediamine-hydroxyethyltriacetate, etc.

As the lower alcohols, there can, for example, be mentioned methanol, ethanol, propanol, isopropanol, isobutyl alcohol, t-butyl alcohol, etc.

As the polyhydric alcohols, there can, for example, be mentioned dihydric alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol and octylene glycol; trihydric alcohols such as glycerol, trimethylolpropane and 1,2,6-hexanetriol; tetrahydric alcohols such as pentaerythritol; pentahydric alcohols such as xylitol; hexahydric alcohols such as sorbitol and mannitol; polymers of polyhydric alcohols such as diethylene glycol, dipropylene glycol, triethylene glycol, polypropylene glycol, tetraethylene glycol, diglycerol, polyethylene glycol, triglycerol, tetraglycerol and polyglycerol; alkyl ethers of dihydric alcohols such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether and ethylene glycol dibutyl ether; alkyl ethers of dihydric alcohols such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether and dipropylene glycol butyl ether; ether and esters of dihydric alcohols such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate and propylene glycol monophenyl ether acetate; glycerol monoalkyl ethers such as chimyl alcohol, oleyl glyceryl ether and batyl alcohol; sugar alcohols such as sorbitol, maltitol, maltotriose, mannitol, sucrose, erythritol, glucose, fructose, sugars obtained by amylolysis, maltose, xylitol and alcohols obtained by reducing sugars obtained by amylolysis; tetrahydrofurfuryl alcohol; POE-tetrahydrofurfuryl alcohol; POP butyl ether; POP.POE butyl ether, tripolyoxypropylene glycerol ether; POP glycerol ether; POP glycerol ether phosphate; POP.POE pentaerythritol ether, etc.

As the monosaccharides, there can, for example, be mentioned trioses such as D-glyceraldehyde and dihydroxyacetone; tetraoses such as D-erythrose, D-erythrulose, D-threose and erythritol; pentaoses such as L-arabinose, D-xylose, L-lyxose, D-arabinose, D-ribose, D-ribulose, D-xylulose and L-xylulose; hexaoses such as D-glucose, D-talose, D-psicose, D-galactose, D-fructose, L-galactose, L-mannose and D-tagatose; heptaoses such as heptose; octaoses such as octose; deoxysugars such as 2-deoxy-D-ribose, 6-deoxy-L-galactose and 6-deoxy-L-mannose; amino sugars such as D-glucosamine, D-galctosamine, sialic acid, aminouronic acids and muramic acid; uronic acids such as D-glucuronic acid, D-mannuronic acid, L-guluronic acid, D-galacturonic acid and L-iduronic acid; etc.

As the oligosaccharides, there can, for example, be mentioned sucrose, gentianose, umbelliferose, lactose, planteose, isolyxose, α,α-trehalose, raffinose, lyxose, stachyose, verbascose, etc.

As the polysaccharides, there can, for example, be mentioned cellulose, quince seed, chondroitin sulfate, starch, galactan, dermatan sulfate, glycogen, gum arabic, heparan sulfate, hyaluronic acid, tragacanth gum, keratan sulfate, chondroitin, xanthane gum, mucoitin sulfuric acid, guar gum, dextran, Locust bean gum, succinoglucans , charonin sulfate, etc.

As the amino acids, there can, for example, be mentioned neutral amino acids such as threonine and cysteine; and basic amino acids such as hydroxylysine. As amino acid derivatives, there can, for example, be mentioned sodium acylsarcosine (sodium lauroylsarcosine), acylglutamate salts, sodium acyl-β-alanine, glutathione, pyrrolidonecarboxylic, etc.

As the organic amines, there can, for example, be mentioned monoethanolamine, diethanolamine, triethanolamine, morpholine, triisopropanolamine, 2-amino-2-methyl-1,3-propanediol, 2-amino-2-methyl-1-propanol, etc.

As the synthetic resin emulsions, there can, for example, be mentioned acrylic resin emulsions, polyethyl acrylate emulsion, acrylic resin solution, polyalkyl acrylate emulsion, polyvinyl acetate emulsion, etc.

As the pH-adjusting agents, there can, for example, be mentioned buffers such as lactic acid-sodium lactate and citric acid-sodium citrate.

As the vitamins, there can, for example, be mentioned vitamins A, B1, B2, B6 and E and their derivatives, pantothenic acid and its derivatives, biotin, etc.

As the antioxidants, there can, for example, be mentioned tocopherols, dibutylhydroxytoluene, butylhydroxyanisole, gallic esters, etc.

As the antioxidant's assistants, there can, for example, be mentioned phosphoric acid, citric acid, ascorbic acid, maleic acid, malonic acid, succinic acid, fumaric acid, cephalin, hexametaphosphate, phytic acid, ethylenediaminetetraacetic acid, etc.

As other compoundable ingredients, there can be mentioned antiseptics such as ethylparaben and butylparaben; antiinflammatory agents such glycyrrhizinic acid derivatives, glycyrrhetinic acid derivatives, salicylic acid derivatives, hinokitiol, zinc oxide and allantoin; whitening agents such as placenta extract and saxifrage extract; extracts of phellodendron bark, Japanese coptis, lithospermum root, herbaceous peony, Japanese chirata, birch, sage, loquat, ginseng, aloe, mallow, iris, grape, coix seed, dishcloth gourd, lily, crocus, cnidium, ginger, Saint-John's-wort, Ononis, garlic, Japanese chillies, dried orange peel, ligusticum root, seaweeds, etc.; activators such as royal jelly, photosensitizing dyes, cholesterol derivatives and infant blood extract; blood circulation accelerators such as benzyl nicotinate, β -butoxyethyl nicotinate, capsaicine, zingerone, cantharis tincture, ichthammol, tannic acid, α-borneol, tocopherol nicotinate, inositol hexanicotinate, cyclandelate, cinnarizine, tolazoline, acetylcholine, verapamil, cepharanthine and γ-oryzanol; antiseborrheics such as sulfur and thianthol; tranexamic acid; thiotaurine; hypotaurine; etc.

For preparing the water-in-oil emulsion preparation for external use on the skin of the invention, special means and/or apparatuses are not needed, and conventional methods for obtaining water-in-oil emulsion preparations can be used. Giving an example, an ester compound, an ester oil, a surfactant and an oil other than ester oil are heated to give a solution, and, if needed, oil soluble or lipophilic physical properties, etc.-improving agents such as an antiseptic and an antioxidant are added, and the mixture is heated to 60 to 80°C to prepare an oil phase. A water soluble inorganic salt, and, if needed, water soluble or hydrophilic physical properties, etc.-improving agents such as a humectant are added to purified water, and the mixture is heated to 60 to 80°C to prepare an aqueous phase. The aqueous phase is gradually added to the oil phase to make preliminary emulsification, and the emulsified particles are made uniform using a homomixer, and the emulsion is deaerated, filtered and cooled.

There is no particular limitation about the form of the water-in-oil emulsion preparation for external use on the skin of the invention, and it can take all forms of conventional water-in-oil emulsion preparations for external use on the skin, such as, for example, cream, liquid cream, foundation, base make, rouge, eye shadow, eye liner, eyebrow, mascara, cosmetics for massage, hand cream, ointment, lipsticks containing emulsified water and hair cream.

### EXAMPLES

The invention is further specifically described below according to examples, but not limited only to the following examples. The compounding amounts in the following examples mean % by mass.

Using a conventional method, 400 g each of water-in-oil emulsion creams having the compounding compositions shown in Tables 2 to 4 were prepared, and evaluation was made on their moisture-confining properties, use touch and stability.

### Test method for moisture-confining properties

For evaluating the moisture-confining properties of oil film formed by the preparation of the invention, the hereinbefore described test of moisture permeation amount was carried out. Since the method for forming oil film on filter paper is somewhat different from that mentioned above, it is described below. A water-in-oil emulsion cream (about 2 g; about 5 g only in Comparative example 10) is uniformly spread on filter paper of 5C, and the resulting filter paper is allowed to stand for 20 hours in a thermo-hygrostat under a temperature of 40°C and a humidity of 30% to evaporate the moisture. Excess oil, etc. on the filter paper is wiped away with a piece of cloth to adjust the impregnated amount to 7 to 9 mg/cm². Measurement was made in a way similar to the already mentioned test method for moisture permeation amount to obtain moisture permeability. Moisture-confining properties were evaluated according to the following judgment criterion, and the results are shown in Tables 5 to 7.

### Judgment criterion for moisture-confining properties

⊚: moisture permeability is less than 7.5%, ○: moisture permeability is 7.5% or more, but less than 10.0%, Δ: moisture permeability is 10.0% or more, but less than 15.0%, ×: moisture permeability is 15.0% or more

### Test method for use touch

An expert panel composed of 10 members actually used the prepared water-in-oil emulsion creams and organoleptically evaluated their use touch. Use touch was evaluated according to the following judgment criterion, and the results are also shown in Tables 5 to 7.

### Judgment criterion for use touch

⊚: 8 members or more of the 10 members judged that the cream is free from stickiness and has good use touch, ○: 6 members or more of the 10 members judged that the cream is free from stickiness and has good use touch, Δ: 4 members or more of the 10 members judged that the cream is free from stickiness and has good use touch, ×: less than 4 members of the 10 members judged that the cream is free from stickiness and has good use touch,

### Test method for stability

The prepared water-in-oil emulsion cream was packed into a glass bottle, and evaluation was made on its stability immediately after the preparation and after it was allowed to stand for one month at 5°C, room temperature or 40°C. Stability was evaluated according to the following judgment criterion, and the results are also shown in Tables 5 to 7.

### Judgment criterion for stability

○: abnormalities such as separation is not observed at all and stability is good,
Δ: slight separation or the like is observed in the oil phase or the aqueous phase,
×: considerable separation is observed in the oil phase or the aqueous phase and stability is poor

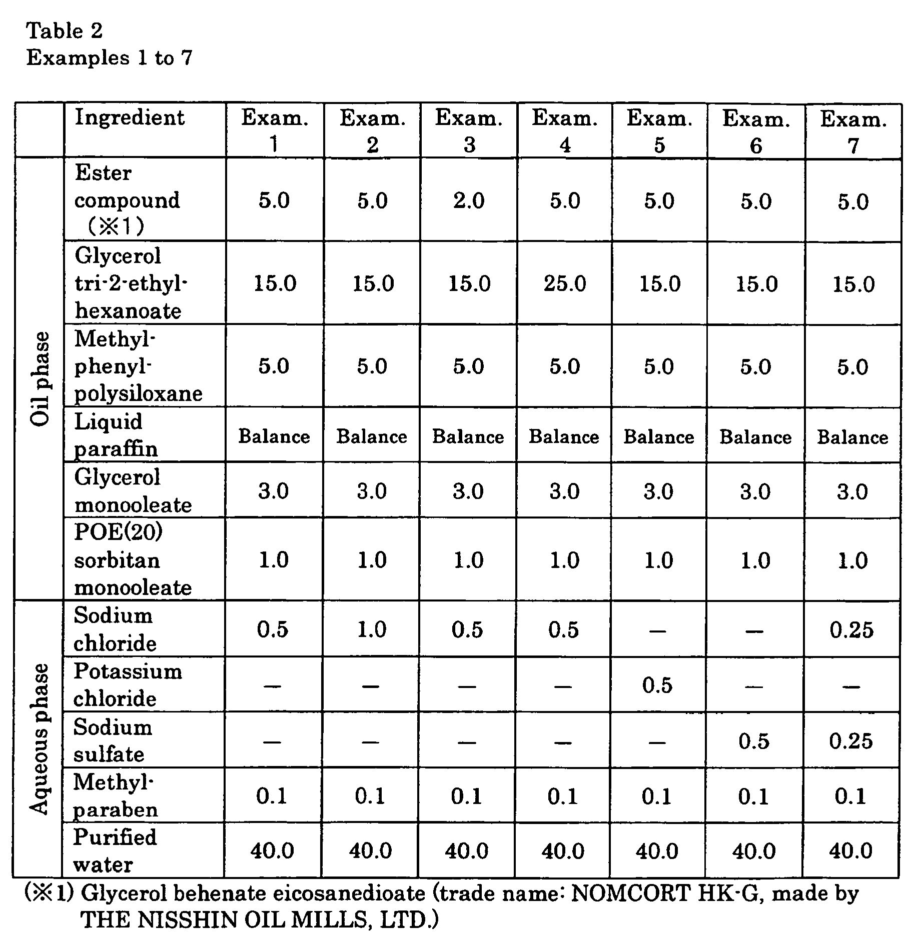

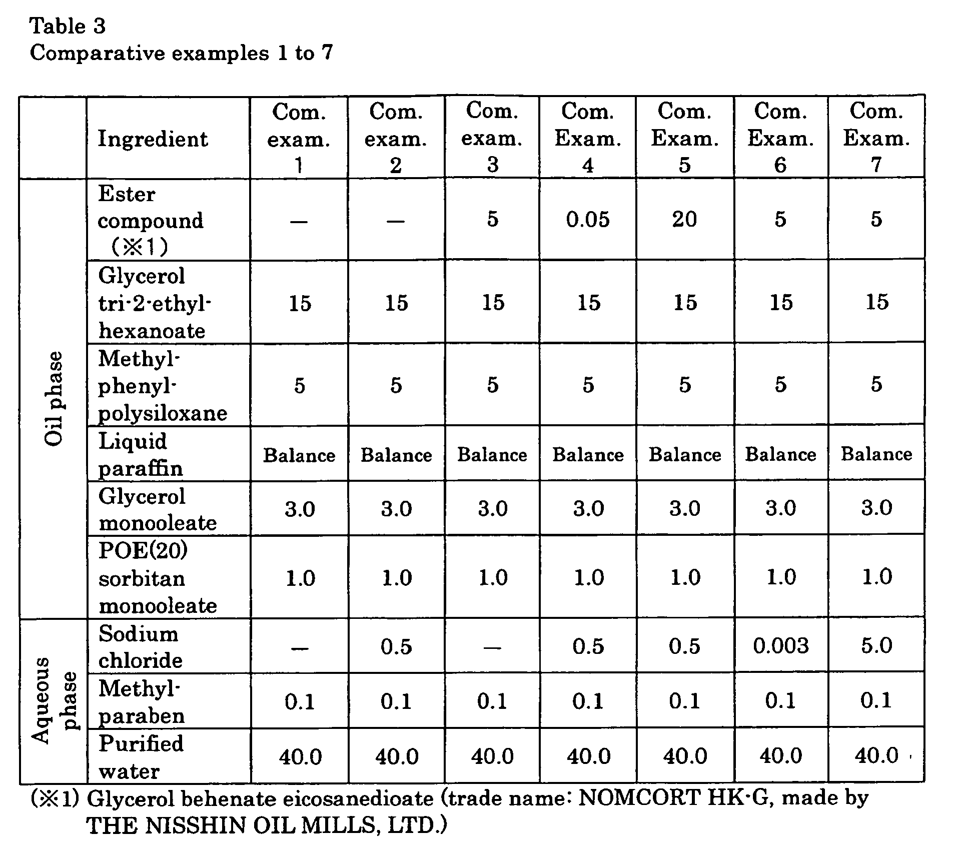

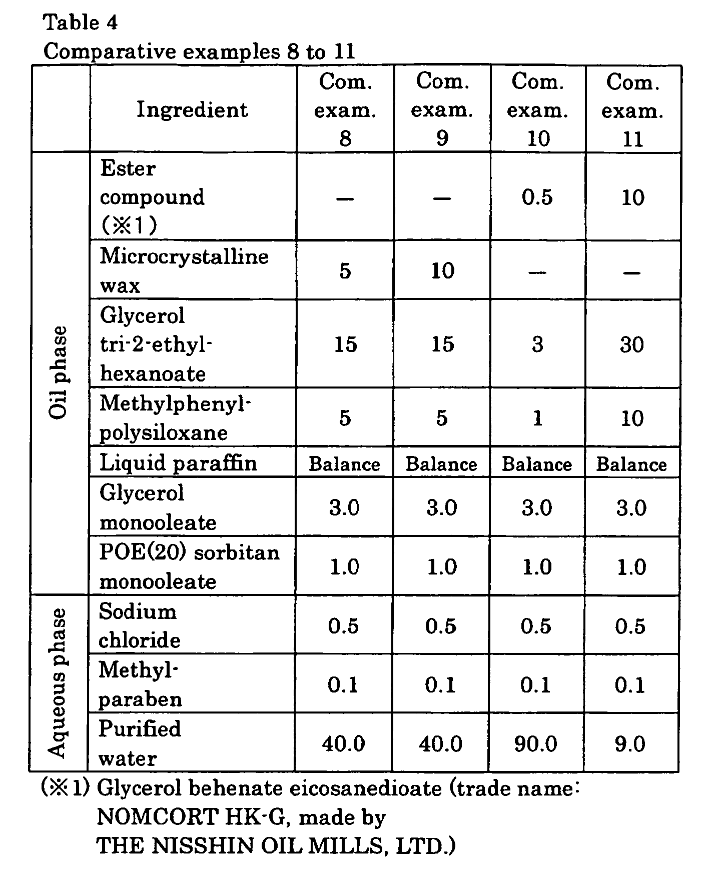

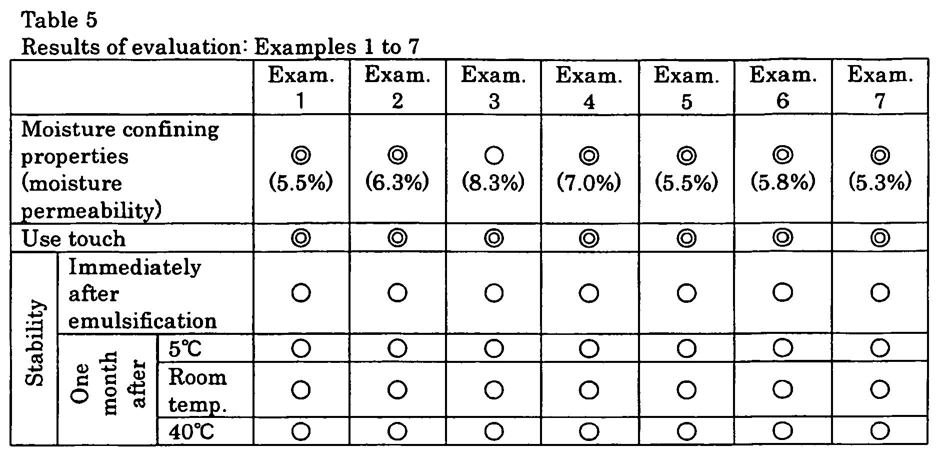

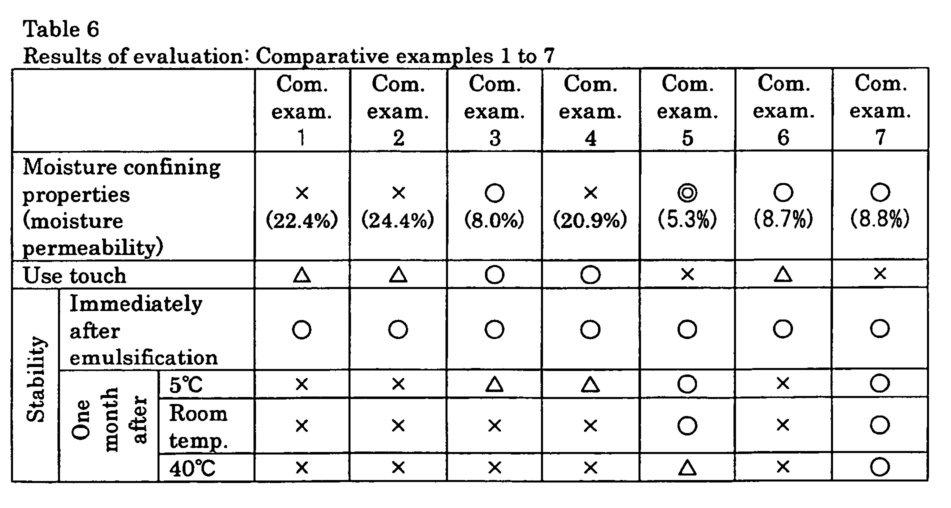

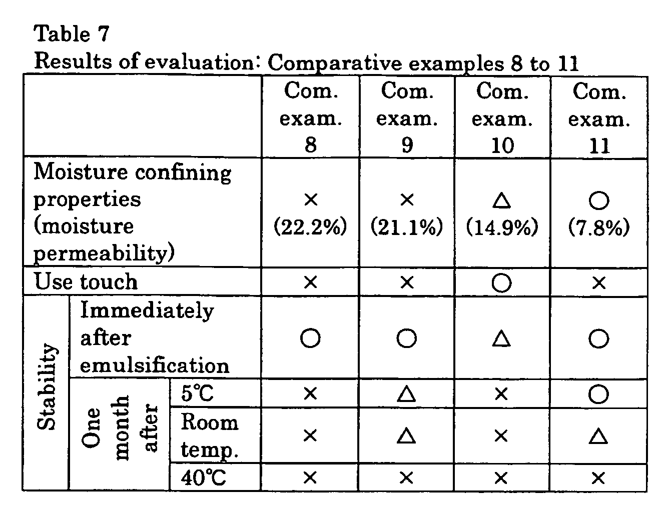

As apparent from Tables 5 to 7, the water-in-oil emulsion creams of Examples 1 to 7 are much superior to those of Comparative examples in moisture-confining properties, use touch and/or stability. Moreover, when the hand coated with any one of the creams of Examples 1 to 7 touched clothes or paper, almost all the part of the oil ingredients did not move thereon, and the creams brought about very good use touch. In the water-in-oil emulsion creams of Comparative examples 1 to 3, both or one of the ester compound and the water soluble inorganic salt, which are indispensable ingredients in the invention, were/was not added, and any of the creams did not satisfy moisture-confining properties, and, in addition, had a problem in stability. In the water-in-oil emulsion creams of Comparative examples 4 to7, the compounding amount of one of the indispensable ingredients of the invention is out of the necessary range, and when the compounding amount is lower than the lower limit of the necessary range, both of moisture-confining properties and stability were poor (Comparative examples 4 and 6), and when the compounding amount is higher than the upper limit of the necessary range, use touch was poor (Comparative examples 5 and 7). Separately, it was tried to stabilize the emulsified state with microcrystalline wax which has so far been used, but, for heightening the stability, it got necessary to compound a large amount of microcrystalline wax, and the resulting water-in-oil emulsion creams not only had strong stickiness, but slid on the skin and easily moved on clothes, etc., and were, thus, low in evaluation of use touch (Comparative examples 8 and 9). Further, in Comparative examples 10 and 11, water-in-oil emulsion creams in which the proportion of the aqueous phase is more than 90% by mass or less than 10% by mass were prepared, but in the case of more than 90% by mass, stability was poor, and in the case of less than 10% by mass, stickiness was strong and thus use touch was poor.

As apparent from Tables 5 to 7, the water-in-oil emulsion creams of Examples 1 to 7 are excellent in moisture-confining properties, and rough skin-improving effect by them can be expected. Actually, 10 housewives who had done a lot of kitchen work were asked to apply the cream of Example 1 on their hands every day before they go to bed over one week, and, except for that, live as usual, and they did that, and, as a result, 8 of them felt that they came to less worry about rough skin than before.

Other examples of the water-in-oil emulsion preparation for external use on the skin of the invention are given below.

### Example 8 Emollient cream

| | |
|---|---|
| Glycerol behenate eicosanedioate (trade name: NOMCORT HK-G, made by THE NISSHIN OIL MILLS, LTD.) | 5 |
| Diglycerol diisostearate | 5 |
| Liquid paraffin | 20 |
| Isooctyl palmitate | 15 |
| Methylphenylpolysiloxane | 5 |
| Glycerol | 5 |
| 1,3-Butylene glycol | 5 |
| 1% Citrate buffer solution (citric acid 1, sodium citrate 9) | 30 |
| Sodium chloride | 0.2 |
| Disodium hydrogenphosphate | 0.1 |
| Hyaluronic acid | 0.1 |
| Carboxymethylcellulose | 0.5 |
| Antiseptic | appropriate amount |
| Perfume | appropriate amount |
| Deionized water | balance |

The emollient cream obtained in Example 8 showed good results in the same evaluation of moisture-confining properties, use touch and stability as made in Examples 1 to 7, namely of moisture-confining properties ⊚, use touch ⊚ and stability (5°C: ○, room temperature: ○, 40°C: ○). Further, when this emollient cream was applied repeatedly onto the part where rough skin had been on one's mind, the rough skin was effectively improved.

### Example 9 Water-in-oil emulsion ointment

| | |
|---|---|
| Glycerol behenate eicosanedioate (trade name: NOMCORT HK-G, made by THE NISSHIN OIL MILLS, LTD.) | 5 |
| Polyoxyethylenepolyalkylsiloxane | 0.5 |
| Glycerol trioctanoate | 20 |
| Corn oil | 20 |
| Paraffin wax | 1 |
| Sorbitol | 5 |
| 1,3-Butylene glycol | 5 |
| Dipotassium glycyrrhizinate | 0.2 |
| Urea | 10 |
| Sodium chloride | 0.1 |
| Sodium sulfate | 0.1 |
| Antiseptic | appropriate amount |
| Purified water | balance |

The water-in-oil emulsion ointment obtained in Example 9 showed good results in the same evaluation of moisture-confining properties, use touch and stability as made in Examples 1 to 7, namely of moisture-confining properties ⊚, use touch ⊚ and stability (5°C: ○, room temperature: ○, 40°C: ○). Further, when this water-in-oil emulsion ointment was applied repeatedly onto the part where rough skin had been on one's mind, the rough skin was effectively improved.

### Referential example 1 Liquid cream

| | |
|---|---|
| Glycerol behenate eicosanedioate (trade name: NOMCORT HK-G, made by THE NISSHIN OIL MILLS, LTD.) | 1 |
| Polyoxyethylenepolyalkylsiloxane | 0.2 |
| Glycerol tri-2-ethylhexanoate | 10 |
| Liquid paraffin | 15 |
| Squalane | 10 |
| Decamethylcyclopentasiloxane | 10 |
| Methylphenylpolysiloxane | 10 |
| Stearyl glycyrrhetinate | 0.1 |
| Sorbitan sesquiolate | 4 |
| POE (20) sorbitan monooleate | 1 |
| Propylene glycol | 8 |
| Glycerol | 5 |
| Potassium chloride | 0.5 |
| Dipotassium hydrogenphosphate | 0.5 |
| Antiseptic | appropriate amount |
| Perfume | appropriate amount |
| Deionized water | balance |

The liquid cream obtained in referential example 1 showed good results in the same evaluation of moisture-confining properties, use touch and stability as made in Examples 1 to 7, namely of moisture-confining properties ⊚, use touch ⊚ and stability (5°C ○, room temperature: ○, 40°C: ○). Further, when this liquid cream was applied repeatedly onto the part where rough skin had been on one's mind, the rough skin was effectively improved.

### Example 10 Sunscreen cream

| | |
|---|---|
| Glycerol behenate eicosanedioate (trade name: NOMCORT HK-G, made by THE NISSHIN OIL MILLS, LTD.) | 4 |
| 2-Ethylhexyl p-methoxycinnamate | 10 |
| 4-Methoxy-4'-t-butylbenzoylmethane | 2 |
| Glycerol tri-2-ethylhexanoate | 10 |
| Squalane | 10 |
| Octamethylcyclotetrasiloxane | 5 |
| Tocopherol acetate | 0.1 |
| Glycerol monooleate | 3.5 |
| POE (20) sorbitan monooleate | 1 |
| Titanium dioxide fine particles made hydrophobic | 3 |
| Zinc oxide fine particles made hydrophobic | 3 |
| 1,3-Butylene glycol | 10 |
| EDTA.2Na | 0.1 |
| Sodium chloride | 1.0 |
| Antiseptic | appropriate amount |
| Perfume | appropriate amount |
| Deionized water | balance |

The sunscreen cream obtained in Example 10 showed good results in the same evaluation of moisture-confining properties, use touch and stability as made in Examples 1 to 7, namely of moisture-confining properties ⊚, use touch ⊚ and stability (5°C: ○, room temperature: ○, 40°C: ○). Further, this sunscreen cream had good affinity to the skin, and when the skin coated with the sunscreen cream touched clothes, the powders did not move thereon, and its use touch was very satisfactory. Furthermore, although when an ester oil as an ultraviolet absorber is compounded, it has been difficult to realize good use touch due to good affinity to the skin and emulsion stability, in Example 10 it was possible to easily compound the ester oil as an ultraviolet absorber.

### Example 11 W/O emulsion foundation

| | |
|---|---|
| Glycerol behenate eicosanedioate (trade name: NOMCORT HK-G, made by THE NISSHIN OIL MILLS, LTD.) | 0.2 |
| Liquid paraffin | 5 |
| Methylphenypolysiloxane | 5 |
| Glycerol tri-2-ethylhexanoate | 5 |
| Polyoxyethylene-modified dimethyl polysiloxane | 4 |
| Sericite | 5 |
| Kaolin | 3 |
| Titanium dioxide | 12 |
| Red iron oxide | 0.5 |
| Yellow iron oxide | 1 |
| Black iron oxide | 0.1 |
| 1,3-Butylene glycol | 5 |
| Sodium sulfate | 0.2 |
| Dispersant | appropriate amount |
| Antiseptic | appropriate amount |
| Perfume | appropriate amount |
| Deionized water | balance |

The W/O emulsion foundation obtained in Example 11 showed good results in the same evaluation of moisture-confining properties, use touch and stability as made in Examples 1 to 7, namely of moisture-confining properties ⊚, use touch ⊚ and stability (5°C: ○, room temperature: ○, 40°C ○). Furthermore, this W/O emulsion foundation had good affinity to the skin, and when the skin coated with the W/O emulsion foundation touched clothes, the powders did not move thereon, and its use touch was very satisfactory

According to the invention, by incorporating a specific ester compound in the oil phase and incorporating a water soluble inorganic salt in the aqueous phase, it is possible to provide a water-in-oil emulsion preparation for external use on the skin which, even when a polar oil such as an ester oil is used as a main ingredient of the oil phase, is excellent in moisture-confining properties, has good use touch, and has good affinity to the skin, thereby there being almost no movement of the oil ingredients onto clothes, etc., and is excellent in emulsion stability, and is usable for general purposes.

## Claims

1. A water-in-oil emulsion preparation for external use on the skin imparting moisture-confining properties, use touch and emulsion stability, wherein
the oil phase comprises a mixture of an ester compound obtained by esterifying (A) a polyhydric alcohol, (B) a fatty acid and/or hydroxy fatty acid each having 8 to 30 carbon atoms and (C) a dibasic carboxylic acid having 12 to 30 carbon atoms; an ester oil; a surfactant; and an oil other than ester oil and the aqueous phase comprises an aqueous solution of a water-soluble inorganic salt
the compounding amounts of the ester oil in the oil phase is 20 to 75 % by mass based on the total mass of the ester compound, the ester oil, the surfactant and the oil other than ester oil, and the water-soluble inorganic salt is at least one selected from sodium chloride, potassium chloride, magnesium chloride, sodium sulfate, sodium phosphate, disodium hydrogenphosphate, sodium dihydrogenphosphate, potassium phosphate, dipotassium hydrogenphosphate and potassium dihydrogenphosphate.

2. The preparation according to claim 1 wherein the mixing ratio in the esterificaton is such that the ratio of ingredient (A): ingredient (B): ingredient (C) is 1.0 mole : 1.0 to 2.5 moles : 0.25 to 1.0 mole.

3. The preparation according to claim 1 or 2, wherein the ratio by mass of the oil phase to the aqueous phase is 10:90 to 90:10.

4. The preparation according to any one of claims 1 to 3, wherein the ester oil is selected from isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, octyldodecyl lactate, lanolin acetate, isocetyl stearate, isocetyl isostearate, cholesteryl 12-hydroxy-stearate, ethylene glycol di-2-ethylhexanoate, dipentaerythritol fatty acid esters, N-alkyl glycol monoisostearate, neopentyl glycol dicaprate, diisostearyl malate, glycerol di-2-heptylundecanoate, trimethylolpropane tri-2-ethylhexanoate, trimethylolpropane triisostearate, pentaerythritol tetra-2-ethylhexanoate, glycerol tri-2-ethylhexanoate, glycerol tri(caprylate, caprate, myristate and stearate), trimethylolpropane triisostearate, cetyl 2-ethylhexanoate, 2-ethylhexyl palmitate, glycerol trimyristate, glycerol tri-2-heptylundecanoate, methyl esters of castor oil fatty acids, oleyl oleate, glycerol acetate, 2-heptylundecyl palmitate, diisobutyl adipate, (adipic acid, 2-ethylhexanoic acid and stearic acid) oligoesters of glycerol, (2-hexyldecanoic acid and sebacic acid) oligoesters of diglycerol, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, glycerol trioctanoate, glycerol triisopalmitate and triethyl citrate.

5. The preparation according to any one of claims 1 to 4, wherein the surfactant is a synthetic and/or a natural surfactant, and one or two or more selected from anionic surfactants, cationic surfactants, amphoteric surfactants, lipophilic nonionic surfactants and hydrophilic nonionic surfactants.

6. The preparation according to any one of claims 1 to 5, wherein the oil other than ester oil is selected from solid fats and oils, waxes, hydrocarbon oils, higher fatty acids, higher alcohols and silicone oils.

7. The preparation according to any one of claims 1 to 6, wherein ingredient (A) is glycerol, ingredient (B) is behenic acid, and ingredient (C) is eicosanedioic acid.

## Patentansprüche

1. Wasser-in-Öl-Emulsionszubereitung zur äußerlichen Anwendung auf der Haut, welche die Feuchtigkeit haltende Eigenschaften, ein angenehmes Gefühl auf der Haut und Beständigkeit der Emulsion verleiht, wobei
die Ölphase eine Mischung aus einer durch Verestern von (A) einem mehrwertigen Alkohol, (B) einer Fettsäure und/oder Hydroxyfettsäure mit jeweils 8 bis 30 Kohlenstoffatomen und (C) einer zweibasigen Carbonsäure mit 12 bis 30 Kohlenstoffatomen erhaltenen Esterverbindung; einem Esteröl; einem Tensid; und einem anderen Öl als Esteröl umfasst, und die wässrige Phase eine wässrige Lösung eines wasserlöslichen anorganischen Salzes umfasst;
die Compoundiermenge des Esteröls in der Ölphase 20 bis 75 Masse-% bezogen auf die Gesamtmasse der Esterverbindung, des Esteröls, des Tensids und des anderen Öls als Esteröl beträgt und das wasserlösliche anorganische Salz mindestens eines ist, das aus Natriumchlorid, Kaliumchlorid, Magnesiumchlorid, Natriumsulfat, Natriumphosphat, Dinatriumhydrogenphosphat, Natriumdihydrogenphosphat, Kaliumphosphat, Dikaliumhydrogenphosphat und Kaliumdihydrogenphosphat ausgewählt ist.

2. Zubereitung nach Anspruch 1, wobei das Mischungsverhältnis bei der Veresterung dergestalt ist, dass das Verhältnis von Bestandteil (A) : Bestandteil (B) : Bestandteil (C) 1,0 mol: 1,0 bis 2,5 mol: 0,25 bis 1,0 mol beträgt.

3. Zubereitung nach Anspruch 1 oder 2, wobei das Massenverhältnis der Ölphase zu der wässrigen Phase 10:90 bis 90:10 beträgt.

4. Zubereitung nach einem der Ansprüche 1 bis 3, wobei das Esteröl aus Isopropylmyristat, Cetyloctanoat, Octyldodecylmyristat, Isopropylpalmitat, Butylstearat, Hexyllaurat, Myristylmyristat, Decyloleat, Hexyldecyldimethyloctanoat, Cetyllactat, Myristyllactat, Octyldodecyllactat, Lanolinacetat, Isocetylstearat, Isocetylisostearat, Cholesteryl-12-hydroxystearat, Ethylenglycol-di-2-ethylhexanoat, Dipentaerythritolfettsäureestern, N-Alkylglycolmonoisostearat, Neopentylglycoldicaprat, Diisostearylmalat, Glycerin-di-2-heptylundecanoat, Trimethylolpropan-tri-2-ethylhexanoat, Trimethylolpropantriisostearat, Pentaerythritol-tetra-2-ethylhexanoat, Glycerin-tri-2-ethylhexanoat, Glycerin-tri(caprylat, -caprat, -myristat und -stearat), Trimethylolpropantriisostearat, Cetyl-2-ethylhexanoat, 2-Ethylhexylpalmitat, Glycerintrimyristat, Glycerin-tri-2-heptylundecanoat, Methylestern von Rizinusölfettsäuren, Oleyloleat, Glycerinacetat, 2-Heptylundecylpalmitat, Diisobutyladipat, (Adipinsäure-, 2-Ethylhexansäure- und Stearinsäure-)oligoestern von Glycerin, (2-Hexyldecansäure- und Sebacinsäure-)oligoestern von Diglycerin, 2-Octyldodecyl-N-lauroyl-L-glutamat, Di-2-heptylundecyladipat, Ethyllaurat, Di-2-ethylhexylsebacat, 2-Hexyldecylmyristat, 2-Hexyldecylpalmitat, 2-Hexyldecyladipat, Diisopropylsebacat, 2-Ethylhexylsuccinat, Glycerintrioctanoat, Glycerintriisopalmitat und Triethylcitrat ausgewählt ist.

5. Zubereitung nach einem der Ansprüche 1 bis 4, wobei das Tensid ein synthetisches und/oder ein natürliches Tensid ist und eines oder zwei oder mehrere aus anionischen Tensiden, kationischen Tensiden, amphoteren Tensiden, lipophilen nichtionischen Tensiden und hydrophilen nichtionischen Tensiden ausgewählt sind.

6. Zubereitung nach einem der Ansprüche 1 bis 5, wobei das andere Öl als Esteröl aus festen Fetten und Ölen, Wachsen, Kohlenwasserstoffölen, höheren Fettsäuren, höheren Alkoholen und Siliconölen ausgewählt ist.

7. Zubereitung nach einem der Ansprüche 1 bis 6, wobei Bestandteil (A) Glycerin ist, Bestandteil (B) Behensäure ist und Bestandteil (C) Eicosandionsäure ist.

## Revendications

1. Préparation en émulsion eau-dans-huile pour usage externe sur la peau, conférant des propriétés de rétention d'eau, de toucher à l'usage et de stabilité d'émulsion, dans laquelle
la phase huileuse comprend un mélange d'un composé de type ester obtenu par estérification de (A) un alcool polyhydrique, (B) un acide gras et/ou un acide gras hydroxylé ayant chacun de 8 à 30 atomes de carbone et (C) un acide carboxylique dibasique ayant de 12 à 30 atomes de carbone ; un huile de type ester ; un tensioactif ; et une huile autre qu'une huile de type ester, et la phase aqueuse comprend une solution aqueuse d'un sel inorganique soluble dans l'eau,
les proportions de mélange de l'huile de type ester dans la phase huileuse valent de 20 à 75 % en masse par rapport à la masse totale du composé de type ester, de l'huile de type ester, du tensioactif et de l'huile autre que l'huile de type ester, et le sel inorganique soluble dans l'eau est au moins un sel choisi parmi le chlorure de sodium, le chlorure de potassium, le chlorure de magnésium, le sulfate de sodium, le phosphate de sodium, le monohydrogénophosphate de sodium, le dihydrogénophosphate de sodium, le phosphate de potassium, le monohydrogénophosphate de sodium et le dihydrogénophosphate de sodium.

2. Préparation selon la revendication 1, dans laquelle le rapport de mélange dans l'estérification est tel que le rapport composant (A) : composant (B) : composant (C) vaut 1,0 mole : 1,0 à 2,5 moles : 0,25 à 1 mole.

3. Préparation selon la revendication 1 ou 2, dans laquelle le rapport en masse de la phase huileuse à la phase aqueuse vaut de 10:90 à 90:10.

4. Préparation selon l'une quelconque des revendications 1 à 3, dans laquelle l'huile de type ester est choisie parmi le myristate d'isopropyle, l'octanoate de cétyle, le myristate d'octyldodécyle, le palmitate d'isopropyle, le stéarate de butyle, le laurate d'hexyle, le myristate de myristyle, l'oléate de décyle, le diméthyloctanoate d'hexyldécyle, le lactate de cétyle, le lactate de myristyle, le lactate d'octyldodécyle, l'acétate de lanoline, le stéarate d'isocétyle, l'isostéarate d'isocétyle, le 12-hydroxy-stéarate de cholestéryle, le 2-éthylhexanoate d'éthylèneglycol, les esters d'acide gras avec le dipentaérythritol, un mono-isostéarate de N-alkylglycol, le dicaprate de néopentylglycol, le malate de diisostéaryle, le di-2-heptylundécanoate de glycérol, le tri-2-éthylhexanoate de triméthylolpropane, le triisostéarate de triméthylolpropane, le tétra-2-éthylhexanoate de pentaérythritol, le tri-2-éthylhexanoate de glycérol, le tri(caprylate, caprate, myristate et stéarate) de glycérol, le triisostéarate de triméthylolpropane, le 2-éthylhexanoate de cétyle, le palmitate de 2-éthylhexyle, le trimyristate de glycérol, le tri-2-heptyl-undécanoate de glycérol, les esters méthyliques d'acides gras d'huile de ricin, l'oléate d'oléyle, l'acétate de glycérol, le palmitate de 2-heptylundécyle, l'adipate de diisobutyle, des oligoesters (acide adipique, acide 2-éthyl-hexanoïque et acide stéarique) de glycérol, des oligoesters (acide 2-hexyl-décanoïque et acide sébacique) de diglycérol, le N-lauroyl-L-glutamate de 2-octyldodécyle, l'adipate de di-2-heptylundécyle, le laurate d'éthyle, le sébaçate de di-2-éthylhexyle, le myristate de 2-hexyldécyle, le palmitate de 2-hexyldécyle, l'adipate de 2-hexyldécyle, le sébaçate de diisopropyle, le succinate de 2-éthylhexyle, le trioctanoate de glycérol, le triisopalmitate de glycérol et le citrate de triéthyle.

5. Préparation selon l'une quelconque des revendications 1 à 4, dans laquelle le tensioactif est un tensioactif naturel et/ou un tensioactif synthétique, et consiste en un ou plusieurs tensioactif(s) choisi(s) parmi les tensioactifs anioniques, les tensioactifs cationiques, les tensioactifs amphotères, les tensioactifs non ioniques lipophiles et les tensioactifs non ioniques hydrophiles.

6. Préparation selon l'une quelconque des revendications 1 à 5, dans laquelle l'huile autre que l'huile de type ester est choisie parmi des huiles et graisses solides, des cires, des huiles hydrocarbonées, des acides gras supérieurs, des alcools supérieurs et des huiles de silicone.

7. Préparation selon l'une quelconque des revendications 1 à 6, dans laquelle le composant (A) est le glycérol, le composant (B) est l'acide béhénique, et le composant (C) est l'acide eicosanedioïque.
